(19) Europäisches Patentamt
European Patent Office
Office européen des brevets

(11) Veröffentlichungsnummer: **0 285 109 B1**

(12) **EUROPÄISCHE PATENTSCHRIFT**

(45) Veröffentlichungstag der Patentschrift: **16.09.92**

(51) Int. Cl.5: **C07C 233/34**, C07C 231/00

(21) Anmeldenummer: **88105115.5**

(22) Anmeldetag: **29.03.88**

(54) **Verfahren zur Herstellung von 2-Amino-4-acylaminophenylethern.**

(30) Priorität: **02.04.87 CH 1257/87**
**02.04.87 CH 1256/87**

(43) Veröffentlichungstag der Anmeldung:
**05.10.88 Patentblatt 88/40**

(45) Bekanntmachung des Hinweises auf die Patenterteilung:
**16.09.92 Patentblatt 92/38**

(84) Benannte Vertragsstaaten:
**CH DE FR GB IT LI**

(56) Entgegenhaltungen:
**EP-A- 0 007 738**
**EP-A- 0 011 048**
**EP-A- 0 142 788**

(73) Patentinhaber: **CIBA-GEIGY AG**
**Klybeckstrasse 141**
**CH-4002 Basel(CH)**

(72) Erfinder: **Leupin, Peter Arnold, Dr.**
**Holbeinstrasse 1**
**CH-4051 Basel(CH)**
Erfinder: **Sartori, Vittore, Dr.**
**Erlensträsschen 75**
**CH-4125 Riehen(CH)**

(74) Vertreter: **Schwabe - Sandmair - Marx**
**Stuntzstrasse 16**
**W-8000 München 80(DE)**

EP 0 285 109 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Anmeldung betrifft ein Verfahren zur Herstellung von 2-Amino-4-acylaminophenylethern sowie deren Verwendung zur Herstellung von Farbstoffen.

2-Amino-4-acylaminophenylether sind wichtige Zwischenprodukte vor allem für die Herstellung von Farbstoffen, insbesondere Dispersionsfarbstoffen; sie werden, beispielsweise durch Reduktion von 2,4-Dinitrophenylethern zu den entsprechenden 2,4-Diaminophenylethern und anschliessender Acylierung der 2,4-Diaminophenylether erhalten. Eine Darstellungsmethode für die 2,4-Dinitrophenylether besteht in der Alkoxylierung von 2,4-Dinitrochlorbenzol mit Alkoholen in Gegenwart einer Base.

Entscheidend für die Verwendung der 2-Amino-4-acylaminophenylether als Zwischenprodukte für die Farbstoffsynthese ist primär deren Gewinnung in hoher Reinheit und damit zusammenhängend die Selektivität der Einführung der Acylgruppe. Neben den gewünschten 2-Amino-4-acylaminophenylethern treten bislang immer die 2-Acylamino-4-aminophenylether, die 2,4-Bisacylaminophenylether sowie nicht umgesetzte 2,4-Diaminophenylether als störende Nebenprodukte in mehr oder weniger grösserer Menge auf.

Es hat daher nicht an Versuchen gefehlt, die Selektivität der Acylierung zu erhöhen. In der EP-B-7738 wird z.B. die Umsetzung von einem Mol 2,4-Diaminophenylether mit 0,6 bis 1,05 Mol Acylierungsmittel in einem Lösungsmittel aus N-substituierten Amiden niedriger aliphatischer Säuren beschrieben, und die EP-A-0 011 048 beschreibt ein Verfahren zur Herstellung von 3-Amino-4-alkoxyacylaniliden durch Acylierung von 2,4-Diaminophenyläther mit äquivalenten Mengen Acylierungsmittel und Diaminoverbindung.

Es wurde nun überraschenderweise gefunden, dass eine hohe Selektivität bezüglich der Einführung des Acylrestes erreicht werden kann, wenn mit einem Ueberschuss von 2,4-Diaminophenylether in Bezug auf das Acylierungsagens gearbeitet wird. Im Vergleich zur EP-B-7738 liegen die weiteren Vorteile des erfindungsgemässen Verfahrens vor allem in der leichten und vollständigen Abtrennbarkeit der erfindungsgemäss eingesetzten alkoholischen Lösungsmittel anstelle der N-substituierten Amide, verbunden mit der Möglichkeit, die Synthese auch ausgehend von 2,4-Dinitrochlorbenzol und ohne Isolierung einer der Zwischenstufen in demselben Lösungsmittel als Eintopfreaktion durchzuführen. Gegenüber der Arbeitsweise der EP-A-0 011 048 ist eine Ausbeuteverbesserung gegeben.

Gegenstand der Erfindung ist somit ein Verfahren zur Herstellung eines 2-Amino-4-propionylaminophenylethers der Formel (I)

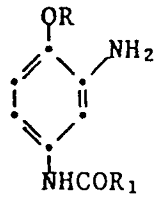

(I)

worin R für Wasserstoff eine $C_1$-$C_4$-Alkylgruppe, Hydroxy-$C_1$-$C_4$-alkylgruppe oder eine $C_1$-$C_4$-Alkoxy-($C_1$-$C_4$)-alkylgruppe und $R_1$ für eine $C_2H_5$-gruppe steht, durch Alkoxylierung von 2,4-Dinitrochlorbenzol zum entsprechenden 2,4-Dinitrophenylether, anschliessender Hydrierung zu den 2,4-Diaminophenylethern und Behandlung der 2,4-Diaminophenylether mit Propionsäureanhydrid, dadurch gekennzeichnet, daß alle Reaktionsschritte in einem niedrigsiedenden alkoholischen Lösungsmittel durchgeführt werden, wobei die entstehenden Zwischenprodukte gegebenenfalls isoliert werden, und die Acylierungsreaktion mit 0,7 bis 0,8 Mol Propionsäureanhydrid pro Mol 2,4-Diaminophenylether durchführt. Die Reaktion kann nicht nur in einem Eintopf-Verfahren ausgehend von 2,4-Dinitrochlorbenzol der Formel

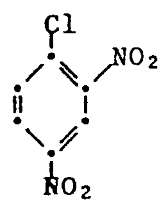

(II)

durchgeführt werden kann, sondern z.B. auch nur in einem einzigen Schritt ausgehend vom 2,4-Diaminophenylether mittels Acylierung. Das in der Acylierungsstufe eingesetzte Acylierungsmittel Propionsäureanhydrid, also bei der Umwandlung der 2,4-Diaminophenylether der Formel III

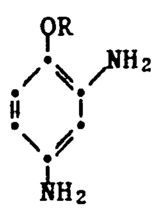

(III)

in die 2-Amino-4-acylaminophenylether der Formel I, wird im molaren Unterschuss von 0,7 bis 0,8 Mol Propionsäureanhydrid, bezogen auf 1 Mol 2,4-Diaminophenylether (III) eingesetzt.

Sowohl das 2,4-Dinitrochlorbenzol, als auch der 2,4-Dinitrophenylether und der 2,4-Diaminophenylether sind bekannte Verbindungen und nach bekannter Art und Weise herstellbar.

Als niedrigsiedende alkoholische Lösungsmittel die im Eintopfverfahren ausgehend vom 2,4-Dinitrochlorbenzol oder nur in der Acylierungsreaktion verwendet werden, kommen vor allem Alkohole mit einem Siedepunkt unter 150 °C, vorzugsweise unter 100 °C, in Frage. Genannt sind beispielsweise: Methanol, Ethanol und die isomeren Propanole und Butanole sowie deren Gemische. Bevorzugt verwendet man Methanol oder Ethanol.

Geht man z.B. von einem 2,4-Diaminophenylether der Formel (III)

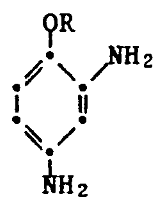

(III)

aus, worin R die genannte Bedeutung hat, so wird dieser Ether mit 0,7 bis 0,8 Mol Propionsäureanhydrid pro Mol 2,4-Diaminophenylether umgesetzt, wobei die Umsetzung in einem niedrigsiedenden alkoholischen Lösungsmittel durchgeführt wird. Der überschüssige 2,4-Diaminophenylether wird dann nach beendeter Reaktion aus dem Reaktionsgemisch abgetrennt.

Vorzugsweise steht für R in den angegebenen Formeln I bis III unabhängig voneinander eine Ethyl-oder Methylgruppe. Insbesondere ist R Methyl. $R_1$ ist Ethyl.

Das Acylierungsmittel für die Acylierungsreaktion ist Propionylanhydrid.

Die Acylierungsreaktion, also die Umsetzung der 2,4-Diaminophenylether der Formel III in die 2-Amino-4-propionylaminophenylether der Formel I, wird bei einer Temperatur von -10° bis 30 °C in einer alkoholischen Lösung gemäss obiger Definition durchgeführt.

In einer bevorzugten Ausführungsform werden 0,8 Mol Propionsäureanhydrid mit einem Mol 2,4-Diaminoanisol in Methanol umgesetzt.

Nach Beendigung der Acylierungsreaktion kann das alkoholische Lösungsmittel leicht und vollständig von der Reaktionsmischung destillativ abgetrennt und wiederverwertet werden.

Anschliessend erfolgt die Entfernung der bei der Umsetzung entstehenden Propionsäure aus dem verbleibenden Gemisch durch Vakuumdestillation.

Der überschüssige 2,4-Diaminophenylether z.B. das 2,4-Diaminoanisol wird durch Vakuum-Rektifikation von der Reaktionsmasse (Schmelze) abgetrennt und kann erneut für die Acylierungsreaktion verwendet werden.

Das so erhaltene Reaktionsgemisch enthaltend den 2-Amino-4-propionylaminophenylether kann dann ohne weitere Reinigung für Farbstoffsynthesen eingesetzt oder gegebenenfalls durch eine weitere Destillation von den vorhandenen Nebenprodukten abgetrennt werden.

Durch diese Art der Aufarbeitung erreicht man eine saubere und nahezu vollständige Abtrennung des Lösungsmittels, der Propionsäure sowie des Diaminophenylethers der Formel III. All diese Komponenten können erneut verwendet werden.

Die folgenden Beispiele veranschaulichen die Erfindung, ohne sie darauf zu beschränken. Prozente sind, sofern nichts anderes vermerkt, Gewichtsprozente.

Beispiel 1:

a) Methoxylierung

202,5 g 2,4-Dinitrochlorbenzol der Formel

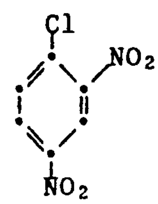

werden als Schmelze vorgelegt und mit 90 g Methanol zu einer Lösung verrührt. 41,9 g Natriumhydroxid werden in 150 g Methanol bei 80-90 °C gelöst, auf 45 °C gekühlt und innert 2 Stunden zur vorgelegten Dinitrochlorbenzollösung zugetropft. Es wird eine Stunde bei Rückflusstemperatur nachgerührt. Die Reaktionssuspension wird unter Druck auf 80 °C erwärmt, wobei das Produkt vollständig in Lösung geht. Durch Filtration wird das entstandene Kochsalz entfernt. Die Lösung enthaltend Dinitroanisol der Formel

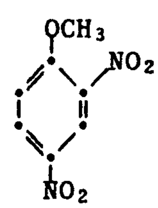

kann direkt für die Folgestufe b) eingesetzt werden.

Ausbeute: 95,0 % der Theorie (LC-Analyse)

Die gleiche Ausbeute wird erhalten, wenn das gesamte Methanol vorgelegt und das Natriumhydroxid in fester Form zugegeben wird.

b) Hydrierung

Die heisse Dinitroanisollösung gemäss a) wird mit 0,8 g 5 % Palladium-auf-Kohle-Katalysator bei 80° unter Druck hydriert (0-40 bar), sodann der Katalysator abfiltriert und mit Methanol gewaschen. Die vereinigten Filtrate enthaltend das 2,4-Diaminoanisol der Formel

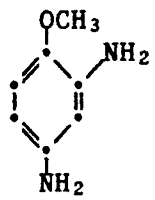

werden direkt für die folgende Stufe c) eingesetzt.
Ausbeute: 99,0 % der Theorie (GC-Analyse, Perchlorsäuretiter)
Die gleiche Ausbeute wird erhalten, wenn anstelle von Palladium/Kohle Raney Nickel als Katalysator eingesetzt wird.

c) Propionylierung

Die Diaminoanisollösung gemäss b) wird auf 0-5°C abgekühlt und bei dieser Temperatur unter intensivem Rühren innert 2 Stunden kontinuierlich mit 104,0 g Propionsäureanhydrid (entsprechend einem Verhältnis von 0,8 Mol Propionsäureanhydrid pro Mol Diaminoanisol) versetzt. Man rührt 1 Stunde nach und lässt die Temperatur auf 25°C ansteigen.

Bei einer Innentemperatur von 65-110°C werden anschliessend Methanol und Wasser abdestilliert. Die gebildete Propionsäure wird im Vakuum abdestilliert. Zur Austreibung der leichtflüchtigen Anteile wird noch 30 Minuten bei 60-100°C im Vakuum nachgerührt. Es werden 188,9 g einer Schmelze mit einer Zusammensetzung von

71,8 % 2-Amino-4-propionylaminoanisol der Formel

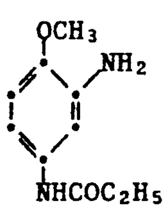

| | |
|---|---|
| 1,5 % | 4-Amino-2-propionylaminoanisol |
| 5,3 % | 2,4-Dipropionylaminoanisol |
| 13,7 % | 2,4-Diaminoanisol |
| 8,1 % | andere Produkte |

erhalten.

Das überschüssige 2,4-Diaminoanisol (ca. 24,0 g) wird durch Vakuum-Rektifikation von der Schmelze abgetrennt und kann wieder im Verfahren gemäss c) eingesetzt werden. Man erhält so nach der Destillation ein Reaktionsgemisch, enthaltend 82,7 % 2-Amino-4-propionylaminoanisol mit einem Nebenproduktanteil an 2,4-Diaminoanisol unter 0,7 %. Das erhaltene 2-Amino-4-propionylaminoanisol kann dann ohne weitere Reinigung als Zwischenprodukt für Farbstoffsynthesen eingesetzt werden.

Unter Berücksichtigung des zurückgewonnenen 2,4-Diaminoanisols liefert die Propionylierung 90,2 % 2-Amino-4-propionylaminoanisol.

Beispiel 2:

a) Methoxylierung

202,5 g 2,4-Dinitrochlorbenzol werden als Schmelze vorgelegt und mit 90 g Methanol zu einer Lösung verrührt. 41,9 g Natriumhydroxid werden in 150 g Methanol bei 80-90°C gelöst, auf 45°C gekühlt und innerhalb von 2 Stunden zur vorgelegten Dinitrochlorbenzol-Lösung zugetropft. Es wird eine Stunde bei Rückflusstemperatur nachgerührt. Aus der Reaktionssuspension werden etwa zwei Drittel des Lösungsmittels abdestilliert. Während der Destillation des restlichen Drittels wird kontinuierlich Wasser zugetropft. Dabei steigt die Temperatur der Reaktionsmasse. Ab ca. 95°C bildet sich eine Emulsion bestehend aus geschmolzener Produktphase und Wasserphase. Letztere enthält das Kochsalz sowie organische Nebenprodukte (überwiegend 2,4-Dinitrophenol).
Nach der Phasentrennung wird die Produktschmelze in Methanol gelöst und in den Folgestufen weiterverarbeitet. Dabei kann das Lösungsmittel der ersten Stufe wiederverwendet werden.
Der Waschprozess kann gegebenenfalls mehrfach durchgeführt werden.
Durch dieses Verfahren wird 2,4-Dinitroanisol in hoher Reinheit erhalten (> 99 % nach LC-Analyse).

b) Hydrierung

Es wird wie in Beispiel 1b) verfahren.

c) Propionylierung

Es wird wie in Beispiel 1c) verfahren, jedoch bei einer Reaktionstemperatur von -5°C und mit einem Verhältnis von 0,7 Mol Propionsäureanhydrid pro Mol Diaminoanisol.
Man erhält ein Reaktionsgemisch folgender Zusammensetzung:

| | |
|---|---|
| 91,6 % | 2-Amino-4-propionylaminoanisol |
| 2,5 % | 4-Amino-2-propionylaminoanisol |
| 5,2 % | 2,4-Dipropionylaminoanisol |
| 0,7 % | sonstige Nebenprodukte |

LC-Analyse (nach Abtrennung von nicht umgesetzten Diaminoanisol).

## Patentansprüche

**1.** Verfahren zur Herstellung eines 2-Amino-4-propionylaminophenylethers der Formel (I)

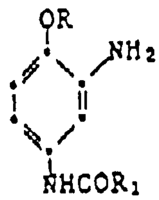

worin R für Wasserstoff, eine $C_1$-$C_4$-Alkylgruppe, Hydroxy-$C_1$-$C_4$-alkylgruppe oder eine $C_1$-$C_4$-Alkoxy-($C_1$-$C_4$)-alkylgruppe und $R_1$ eine $C_2H_5$-Gruppe steht, durch Alkoxylierung von 2,4-Dinitrochlorbenzol zum entsprechenden 2,4-Dinitrophenylether, anschließender Hydrierung zu den 2,4-Diaminophenylethern und Behandlung der 2,4-Diaminophenylether mit Propionsäureanhydrid, dadurch gekennzeichnet, daß man alle Reaktionsschritte in einem niedrigsiedenden alkoholischen Lösungsmittel durchführt, wobei die entstehenden Zwischenprodukte gegebenenfalls isoliert werden können, und die Acylierungsreaktion mit 0,7 bis 0,8 Mol Propionsäureanhydrid pro Mol 2,4-Diaminophenylether durchführt.

**2.** Verfahren nach Anspruch 1, dadurch gekennzeichnet, daß eines oder mehrere der entstehenden Zwischenprodukte isoliert wird/werden.

**3.** Verfahren gemäß Anspruch 1, dadurch gekennzeichnet, daß man als Lösungsmittel Methanol, Ethanol, Propanol, Isopropanol, Butanol oder ein Gemisch verwendet.

**4.** Verfahren gemäß Anspruch 3, dadurch gekennzeichnet, daß man als Lösungsmittel Methanol oder Ethanol verwendet.

**5.** Verfahren gemäß Anspruch 1, dadurch gekennzeichnet, daß man zur Herstellung eines 2-Amino-4-propionylaminophenylethers von einem 2,4-Diaminophenylether der Formel (III)

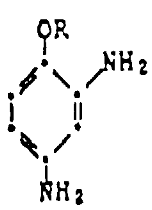

worin R die im Anspruch 1 angegebene Bedeutung hat, ausgeht und diesen mit 0,7 bis 0,8 Mol Propionsäureanhydrid pro Mol 2,4-Diaminophenylether umsetzt, und der überschüssige 2,4-Diaminophenylether nach beendeter Reaktion aus dem Reaktionsgemisch abgetrennt wird.

**6.** Verfahren nach Anspruch 1, dadurch gekennzeichnet, daß in einem Eintopfverfahren 2-Amino-4-propionylaminophenylether ausgehend von 2,4-Dinitrochlorbenzol hergestellt wird.

**7.** Verfahren gemäß einem der Ansprüche 1 bis 6, dadurch gekennzeichnet, daß man als 2,4-Diaminophenylether den Methoxy- oder den Ethoxyether verwendet.

**8.** Verfahren gemäß Anspruch 7, dadurch gekennzeichnet, daß man 2,4-Diaminoanisol verwendet.

**9.** Verfahren gemäß Anspruch 8, dadurch gekennzeichnet, daß 0,8 Mol Propionsäureanhydrid mit einem Mol 2,4-Diaminoanisol in Methanol umgesetzt werden.

**10.** Verfahren gemäß Anspruch 1, dadurch gekennzeichnet, daß die Reaktion bei einer Temperatur von - 10 bis 30 °C durchgeführt wird.

## Claims

**1.** A process for the preparation of a 2-amino-4-propionylaminophenyl ether of formula (I)

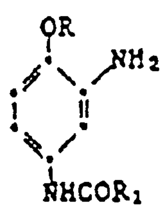

in which R is hydrogen, a $C_1$-$C_4$ alkyl group, a hydroxy-$C_1$-$C_4$ alkyl group or a $C_1$-$C_4$ alkoxy-$C_1$-$C_4$ alkyl group and $R_1$ is a $C_2H_5$ group, by alkoxylating 2,4-dinitrochlorobenzene to the corresponding 2,4-dinitrophenyl ether, subsequently hydrogenating the latter to give a 2,4-diaminophenyl ether and treating the latter with propionic anhydride, which comprises carrying out all the reaction steps in a low-boiling alcoholic solvent, and, if desired, isolating the resultant intermediates, and carrying out the acylation reaction with 0.7 to 0.8 mol of propionic anhydride per mole of 2,4-diaminophenyl ether.

2. A process according to claim 1, wherein one or more of the resultant intermediates is/are isolated.

3. A process according to claim 1, wherein the solvent employed is methanol, ethanol, propanol, isopropanol, butanol or a mixture thereof.

4. A process according to claim 3, wherein the solvent employed is methanol or ethanol.

5. A process according to claim 1, which comprises, for the preparation of a 2-amino-4-propionylaminophenyl ether, starting from a 2,4-diaminophenyl ether of formula (III)

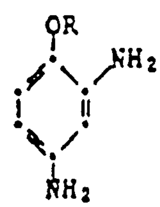

in which R is as defined in claim 1, and reacting said ether with 0.7 to 0.8 mol of propionic anhydride per mole of 2,4-diaminophenyl ether, and separating off the excess 2,4-diaminophenyl ether from the reaction mixture after completion of the reaction.

6. A process according to claim 1, which comprises preparing a 2-amino-4-propionylaminophenyl ether in a one-pot process starting from 2,4-dinitrochlorobenzene.

7. A process according to any one of claims 1 to 6, wherein the 2,4-diaminophenyl ether employed is the methoxy or ethoxy ether.

8. A process according to claim 7, wherein 2,4-diaminoanisole is employed.

9. A process according to claim 8, wherein 0.8 mol of propionic anhydride is reacted with one mole of 2,4-diaminoanisole in methanol.

10. A process according to claim 1, wherein the reaction is carried out at a temperature from -10 to 30 °C.

**Revendications**

1. Procédé de préparation d'un éther de 2-amino-4-propionylaminophényle de formule (I)

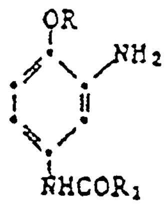

dans laquelle R représente un atome d'hydrogène, un groupe alkyle en $C_{1-4}$, un groupe hydroxyalkyle en $C_{1-4}$ ou un groupe alcoxy-($C_{1-4}$)-alkyle($C_{1-4}$), et $R_1$ est un groupe $C_2H_5$, par alcoxylation de 2,4-dinitrochlorobenzène en éther 2,4-dinitrophénylique correspondant, suivie d'une hydrogénation en éther 2,4-diaminophénylique et traitement de l'éther 2,4-diaminophénylique avec l'anhydride d'acide propionique, caractérisé en ce que toutes les étapes réactionnelles sont réalisées dans un solvant alcoolique de bas point d'ébullition, les produits intermédiaires formés pouvant éventuellement être isolés, et en ce qu'on effectue la réaction d'acylation avec de 0,7 à 0,8 mole d'anhydride d'acide propionique par mole d'éther 2,4-diaminophénylique.

2. Procédé selon la revendication 1, caractérisé en ce qu'un ou plusieurs des produits intermédiaires formés est (sont) isolé(s).

3. Procédé selon la revendication 1, caractérisé en ce qu'on utilise, comme solvant, du méthanol, de l'éthanol, du propanol, de l'isopropanol, du butanol, ou un mélange de ces solvants.

4. Procédé selon la revendication 3, caractérisé en ce qu'on utilise, comme solvant, du méthanol ou de l'éthanol.

5. Procédé selon la revendication 1, caractérisé en ce que, pour préparer un éther de 2-amino-4-propionylaminophényle, on part d'un éther 2,4-diaminophénylique de formule (III)

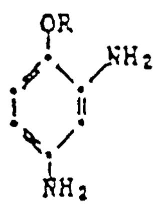

dans laquelle R a la définition indiquée dans la

revendication 1, et on fait réagir ce dernier avec de 0,7 à 0,8 mole d'anhydride d'acide propionique par mole d'éther 2,4-diaminophénylique, puis, une fois la réaction terminée, on sépare d'avec le mélange réactionnel l'excès d'éther 2,4-diaminophénylique.

**6.** Procédé selon la revendication 1, caractérisé en ce qu'on prépare, à l'aide d'une réaction en récipient unique, un éther de 2-amino-4-propionylaminophényle à partir de 2,4-dinitrochlorobenzène.

**7.** Procédé selon une des revendications 1 à 6, caractérisé en ce qu'on utilise, comme éther 2,4-diaminophénylique, l'éther méthoxy ou l'éther éthoxy.

**8.** Procédé selon la revendication 7, caractérisé en ce qu'on utilise le 2,4-diaminoanisole.

**9.** Procédé selon la revendication 8, caractérisé en ce qu'on fait réagir 0,8 mole d'anhydride d'acide propionique avec 1 mole de 2,4-diaminoanisole dans du méthanol.

**10.** Procédé selon la revendication 1, caractérisé en ce que la réaction est effectuée à une température de -10 à 30 ° C.